# EUROPEAN PATENT SPECIFICATION

(11) **EP 2 427 052 B1**
(45) Date of publication and mention of the grant of the patent: **26.08.2015**
(21) Application number: 10772657.2
(22) Date of filing: 03.05.2010
(51) Int. Cl.: A61K 31/13, A61P 27/06

(54) **COMPOSITIONS FOR USE IN THE TREATMENT OF GLAUCOMA OR OCULAR HYPERTENSION**
ZUSAMMENSETZUNGEN ZUR VERWENDUNG IN DER BEHANDLUNG VON GLAUKOM ODER AUGENHOCHDRUCK
COMPOSITIONS POUR L'UTILISATION DANS LE TRAITEMENT DU GLAUCOME OU D'HYPERTENSION OCULAIRE

(30) Priority: 04.05.2009 US 175402 P
(43) Date of publication of application: 14.03.2012
(73) Proprietor: Acucela, Inc., Seattle, WA 98101 (US)
(72) Inventor: MATA, Nathan L., San Diego, California 92130 (US); NARAYAN, Sujatha, San Diego, California 92122 (US); TSIVKOVSKAIA, Natalia, San Diego, California 92127 (US)
(74) Representative: Cole, William Gwyn
(86) International application number: PCT/US2010/033441
(87) International publication number: WO 2010/129486

(56) References cited:
- EP-B1- 1 768 657
- WO-A1-2006/007314
- WO-A2-2005/087210
- US-B2- 6 646 001
- MATA N L ET AL: "Effects of N-(4-hydroxyphenyl) retinamide on vitamin A homeostasis and A2E biosynthesis in abcr null mutant mice", IOVS , vol. 46 1 May 2005 (2005-05-01), 5 May 2005 (2005-05-05), XP002680488, & ANNUAL MEETING OF THE ASSOCIATION-FOR-RESEARCH-IN-VISION-AND-OPH THALMOLOGY; FT LAUDERDALE, FL, USA ISSN: 0146-0404 Retrieved from the Internet: URL:http://abstracts.iovs.org/cgi/content/ abstract/46/5/1744 [retrieved on 2012-07-20]
- RADU R A ET AL: "REDUCTIONS IN SERUM VITAMIN A ARREST ACCUMULATION OF TOXIC RETINAL FLUOROPHORES: A POTENTIAL THERAPY FOR TREATMENT OF LIPOFUSCIN-BASED RETINAL DISEASES", INVESTIGATIVE OPHTHALMOLOGY & VISUAL SCIENCE, vol. 46, no. 12, 1 December 2005 (2005-12-01), pages 4393-4401, XP009064844, ASSOCIATION FOR RESEARCH IN VISION AND OPHTHALMOLOGY, US ISSN: 0146-0404, DOI: 10.1167/IOVS.05-0820
- MAEDA AKIKO ET AL: "Effects of potent inhibitors of the retinoid cycle on visual function and photoreceptor protection from light damage in mice", MOLECULAR PHARMACOLOGY, vol. 70, no. 4, October 2006 (2006-10), pages 1220-1229 URL, XP002680489, ISSN: 0026-895X
- SREEKUMAR PARAMESWARAN G ET AL: "N-(4-hydroxyphenyl) retinamide augments laser-induced choroidal neovascularization in mice", IOVS, vol. 49, no. 3, March 2008 (2008-03), pages 1210-1220, XP002680490, ISSN: 0146-0404

## Description

### CROSS-REFERENCE

This application claims the benefit of U.S. Provisional Application No. 61/175,402, filed May 4, 2009.

### FIELD OF THE INVENTION

The methods and compositions described herein are directed to the treatment of ophthalmic conditions.

### BACKGROUND OF THE INVENTION

The human eye is an important human sense organ. It allows humans conscious light perception, vision, which includes color differentiation, and the perception of depth. Diseases of the eye associated with angiogenesis and/or damage to ganglia can ultimately lead to blindness in the affected individual.

### SUMMARY OF THE INVENTION

Presented herein are methods, compositions and formulations for treating ophthalmic conditions associated with at least one of the following symptoms or pathologies: (a) destruction or interruption of the integrity of the ganglion cell layer in the eye; (b) accumulation of advanced glycation end products (AGEs) and their receptors (RAGEs) in the retina; (c) p38 MAPK-mediated cell death in the eye; (d) over expression or accumulation of VEGF in the eye; (e) growth and/or differentiation a retinal microvasculature; (f) corneal neo-vascularization; (g) expression of AGEs/RAGEs in the retina; (h) ocular angiogenesis; (i) ganglion cell death; (j) damage to ganglia; or (k) retinal vascular leakage. In some embodiments, the ophthalmic conditions is associated with at least two of the aforementioned symptoms or pathologies

Also presented herein are methods, compositions and formulations for treating ophthalmic conditions associated with at least one of the following symptoms or pathologies: excessive N-acetyl glucoseamine (GlcNAc) on pericyte membranes; decrease in ceramide; decrease in GM3; and reduction in pericyte proliferation. In certain embodiments, the compositions described herein prevent vascular leakage, including by preserving pericytes; and/or decreasing AGE expression. In certain embodiments, the compositions described herein reduce VEGF activity, including by increasing ceramide signaling; increasing GM3; modulating the glycosphingolipid pathway; and/or increasing sphingosine-1-phosphate. In certain embodiments, the compositions herein downregulate the VEGFR2 promoter.

Also presented herein are methods, compositions and formulations for treating ophthalmic conditions associated with inflammation, including by decreasing IL8 expression, inhibition of NFkB, a reduction in MMP-9, a reduction in cyclooxygenase-2 and/or a reduction in VEGF.

Also presented herein are methods, compositions and formulations for treating ophthalmic conditions associated with the need for neuroprotection, including by protecting retinal ganglion cells, inhibition of NFkB, and/or modulating the PI3K/Akt pathway.

Relevant diseases include glaucoma, ocular hypertension or combination thereof.

In one embodiment, such conditions are treated by administration (including oral administration of an effective amount of a first compound having the structure of Formula (II): 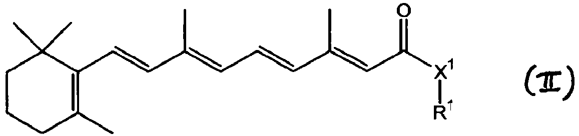 wherein X₁ is selected from the group consisting of NR², O, S, CHR²; R¹ is (CHR²)ₓ-L¹-R³, wherein x is 0, 1, 2, or 3; L¹ is a single bond or -C(O)-; R² is a moiety selected from the group consisting of H, (C₁-C₄)alkyl, F, (C₁-C₄)fluoroalkyl, (C₁-C₄)allcoxy, -C(O)OH, -C(O)-NH₂, -(C₁-C₄)alkylamine, -C(O)-(C₁-C₄)alkyl, -C(O)-(C₁-C₄)fluoroalkyl, -C(O)-(C₁-C₄)alkylamine, and -C(O)-(C₁-C₄)alkoxy; and R³ is H or a moiety, optionally substituted with 1-3 independently selected substituents, selected from the group consisting of (C₂-C₇)alkenyl, (C₂-C₇)alkynyl, aryl, (C₃-C₇)cycloalkyl, (C₅-C₇)cycloalkenyl, and a heterocycle, provided that R³ is not H when both x is 0 and L¹ is a single bond; or a pharmaceutically acceptable salt or solvate thereof.

In any of the aforementioned aspects are further embodiments in which (a) X¹ is NR², wherein R² is H or (C₁-C₄)alkyl; (b) wherein x is 0; (c) x is 1 and L¹ is -C(O)-; (d) R³ is an optionally substituted aryl; (e) R³ is an optionally substituted heteroaryl; (f) X¹ is NH and R³ is an optionally substituted aryl, including yet further embodiments in which (i) the aryl group has one substituent, (ii) the aryl group has one substituent selected from the group consisting of halogen, OH, O(C₁-C₄)alkyl, NH(C₁-C₄)alkyl, O(C₁-C₄)fluoroalkyl, and N[(C₁-C₄)alkyl]₂, (iii) the aryl group has one substituent, which is OH, (v) the aryl is a phenyl, or (vi) the aryl is naphthyl; (g) the compound is 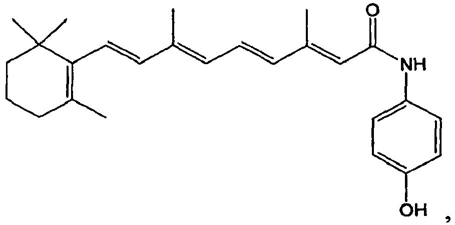 or a pharmaceutically acceptable or solvate thereof; (h) the compound is 4-hydroxyphenylretinamide, or a pharmaceutically acceptable solvate thereof; (i) the compound is 4-methoxyphenylretinamide, or (j) 4-oxo fenretinide, or a pharmaceutically acceptable salt or solvate thereof.

In further embodiment of the pharmaceutical composition aspect, the pharmaceutical composition further comprising an effective amount of at least one additional agent selected from the group consisting of an inducer of nitric oxide production, an anti-inflammatory agent, a physiologically acceptable antioxidant, a physiologically acceptable mineral, a negatively charged phospholipid, a carotenoid, a statin, an anti-angiogenic drug, a matrix metalloproteinase inhibitor, resveratrol and other trans-stilbene compounds, and an agent that inhibits, antagonizes or short-circuits the visual cycle at a step of the visual cycle that occurs outside a disc of a rod photoreceptor cell. In further embodiments, (a) the additional agent is a physiologically acceptable antioxidant; (b) the additional agent is an inducer of nitric oxide production; (c) the additional agent is an anti-inflammatory agent; (d) the additional agent is a physiologically acceptable mineral; (e) the additional agent is a negatively charged phospholipid; (f) the additional agent is a carotenoid; (g) the additional agent is a statin; (h) the additional agent is an anti-angiogenic agent; (i) the additional agent is a matrix metalloproteinase inhibitor; (j) the additional agent is an agent that inhibits, antagonizes or short-circuits the visual cycle at a step of the visual cycle that occurs outside a disc of a rod photoreceptor cell; or (k) resveratrol and other *trans*-stilbene compounds.

Other objects, features and advantages of the methods and compositions described herein will become apparent from the following detailed description. It should be understood, however, that the detailed description and the specific examples, while indicating specific embodiments, are given by way of illustration only, since various changes and modifications within the spirit and scope of the invention will become apparent to those skilled in the art from this detailed description.

### BRIEF DESCRIPTION OF THE FIGURES

Figure 1. An illustrative example providing evidence that HPR treatment does not alter hyperglycemia in *Ins2Akital+* mice.
Figure 2. An illustrative example providing evidence that HPR preserves integrity of the ganglion cell layer.
Figure 3. An illustrative example providing evidence that HPR reduces the accumulation of advanced glycation end products in the retina.
Figure 4. An illustrative example providing evidence that HPR treatment attenuates p38 MAPK-mediated cell death in the RPE.
Figure 5. An illustrative example providing evidence that HPR potently modulates VEGF expression in *Ins2Akital+* mice.
Figure 6. An illustrative example providing evidence that HPR inhibits the growth and differentiation of primary human retinal microvascular endothelial cells (HMRECs) in *the in vitro* capillary tube formation assay.
Figure 7. An illustrative example providing evidence that growth factor-induced corneal neo-vascularization is dramatically reduced in HPR treated mice in *the in vivo* corneal micropocket assay.
Figure 8. An illustrative example providing evidence that HPR treatment results in a significant reduction in the extent of retinal vascular leakage in an animal model of retinal neovascularization.

### DETAILED DESCRIPTION OF THE INVENTION

*N*-(4-hydroxyphenyl)retinamide (HPR) is a synthetic retinoid that halts the production of toxic fluorophores in the retina by reducing serum retinol. HPR is currently in a phase II clinical trial for the treatment of geographic atrophy. While it is well tolerated and has a favorable toxicity profile, the role of HPR on angiogenesis in ocular tissue has not been addressed. Described herein are methods and compositions comprising a compound of Formula (II) (e.g., *N*-(4-hydroxyphenyl)retinamide or *N*-(4-methoxyphenyl)retinamide) for the reduction of retinal pathology and angiogenesis (e.g., including that resulting from hyperglycemic stress).

Macular or Retinal Degenerations and Dystrophies. Macular degeneration (also referred to as retinal degeneration) is a disease of the eye that involves deterioration of the macula, the central portion of the retina. Approximately 85% to 90% of the cases of macular degeneration are the "dry" (atrophic or non-neovascular) type. In dry macular degeneration, the deterioration of the retina is associated with the formation of small yellow deposits, known as drusen, under the macula; in addition, the accumulation of lipofuscin in the RPE leads to photoreceptor degeneration and geographic atrophy. This phenomena leads to a thinning and drying out of the macula. The location and amount of thinning in the retina caused by the drusen directly correlates to the amount of central vision loss. Degeneration of the pigmented layer of the retina and photoreceptors overlying drusen become atrophic and can cause a slow loss of central vision. Ultimately, loss of retinal pigment epithelium and underlying photoreceptor cells results in geographic atrophy. Administration of at least one compound having the structure of Formula (II) to a mammal reduces the formation of, or limit the spread of, photoreceptor degeneration and/or geographic atrophy in the eye of the mammal. By way of example only, administration of HPR and/or MPR to a mammal, are used to treat photoreceptor degeneration and/or geographic atrophy in the eye of the mammal.

In "wet" macular degeneration new blood vessels form (i.e., neovascularization) to improve the blood supply to retinal tissue, specifically beneath the macula, a portion of the retina that is responsible for our sharp central vision. The new vessels are easily damaged and sometimes rupture, causing bleeding and injury to the surrounding tissue. Although wet macular degeneration only occurs in about 10 percent of all macular degeneration cases, it accounts for approximately 90% of macular degeneration-related blindness. Neovascularization can lead to rapid loss of vision and eventual scarring of the retinal tissues and bleeding in the eye. This scar tissue and blood produces a dark, distorted area in the vision, often rendering the eye legally blind. Wet macular degeneration usually starts with distortion in the central field of vision. Straight lines become wavy. Many people with macular degeneration also report having blurred vision and blank spots (scotoma) in their visual field.

Glaucoma is a disease of the optic nerve involving loss of retinal ganglion cells in a characteristic pattern of optic neuropathy. It is a disorder associated with pressure in the eye and is characterized by damage to the optic nerve with consequent visual loss, initially peripheral, but potentially blinding. Although raised intraocular pressure is a significant risk factor for developing glaucoma, there is no set threshold for intraocular pressure that causes glaucoma. Eye pressure, perfusion of the optic nerve, mechanical factors in and around the optic nerve, and biochemical factors also play a role in the pathogenesis of glaucoma. Primary open angle glaucoma (POAG) is the most common of all types of glaucoma. The condition is diagnosed in the presence of an open angle, evidence of optic nerve damage, and peripheral vision loss consistent with glaucoma on a visual field test.

Risk factors for glaucoma include elevated intraocular pressure, family history of glaucoma, advanced age, cardiovascular disease, diabetes mellitus, myopia, and high blood pressure, to name a few. Oxidative damage and lipid peroxidation have also been found to have a role in the pathogenesis of POAG, as measured by elevated levels of plasma MDA in patients with POAG. Yildirim O, Eye 19(5):580-3 (2005).

Other factors that contribute to conditions of the eye caused by oxidative stress or damage can be further caused or exacerbated by, e.g., diabetes, hypertension, arteriosclerosis, macular drusen, or smoking of tobacco.

Untreated glaucoma leads to severe defects in the structure of the eye, particularly to damage of the head of the optic nerve, resulting in reduction of the visual field and optical atrophy. In certain instances, the pathology is related to insufficient drainage of aqueous humor from the eye. Other factors, including the production of aqueous humor and pressure on the episcleral veins, may also contribute to development of the condition.

### CHEMICAL TERMINOLOGY

The term "aromatic" or "aryl" refers to an aromatic group which has at least one ring having a conjugated pi electron system and includes both carbocyclic aryl (*e.g*., phenyl) and heterocyclic aryl (or "heteroaryl" or "heteroaromatic") groups (*e.g*., pyridine). The term includes monocyclic or fused-ring polycyclic (*i.e.*, rings which share adjacent pairs of carbon atoms) groups. The term "carbocyclic" refers to a compound which contains one or more covalently closed ring structures, and that the atoms forming the backbone of the ring are all carbon atoms. The term thus distinguishes carbocyclic from heterocyclic rings in which the ring backbone contains at least one atom which is different from carbon.

The terms "heteroaryl" or, alternatively, "heteroaromatic" refers to an aryl group that includes one or more ring heteroatoms selected from nitrogen, oxygen and sulfur. An *N*-containing "heteroaromatic" or "heteroaryl" moiety refers to an aromatic group in which at least one of the skeletal atoms of the ring is a nitrogen atom. The polycyclic heteroaryl group is optionally fused or non-fused. Illustrative examples of heteroaryl groups include the following moieties: 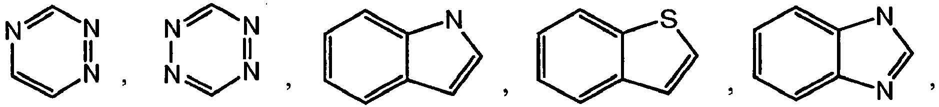 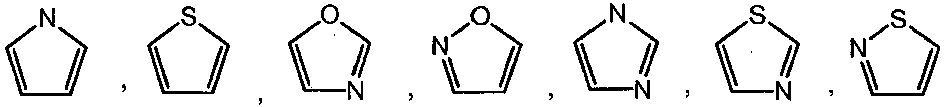 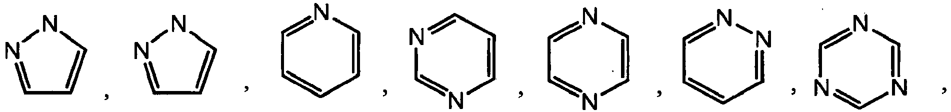 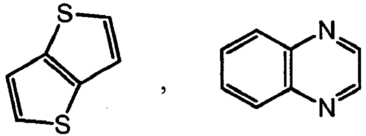 and the like.

The term "heterocycle" refers to heteroaromatic and heteroalicyclic groups containing one to four heteroatoms each selected from O, S and N, wherein each heterocyclic group has from 4 to 10 atoms in its ring system, and with the proviso that the ring of said group does not contain two adjacent O or S atoms. Non-aromatic heterocyclic groups include groups having only 4 atoms in their ring system, but aromatic heterocyclic groups must have at least 5 atoms in their ring system. The heterocyclic groups include benzo-fused ring systems. An example of a 4-membered heterocyclic group is azetidinyl (derived from azetidine). An example of a 5-membered heterocyclic group is thiazolyl. An example of a 6-membered heterocyclic group is pyridyl, and an example of a 10-membered heterocyclic group is quinolinyl. Examples of non-aromatic heterocyclic groups are pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the groups listed above, are optionally C-attached or *N*-attached where such is possible. For instance, a group derived from pyrrole includes pyrrol-1-yl (*N*-attached) or pyrrol-3-yl (C-attached). Further, a group derived from imidazole includesimidazol-1-yl or imidazol-3-yl (both *N*-attached) or imidazol-2-yl, imidazol-4-yl or imidazol-5-yl (all C-attached). The heterocyclic groups include benzo-fused ring systems and ring systems substituted with one or two oxo (=O) moieties such as pyrrolidin-2-one.

A "heteroalicyclic" group refers to a cycloalkyl group that includes at least one heteroatom selected from nitrogen, oxygen and sulfur. The radicals are optionally fused with an aryl or heteroaryl. Illustrative examples of heterocycloalkyl groups include: 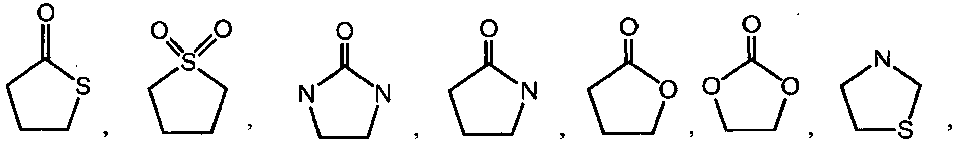 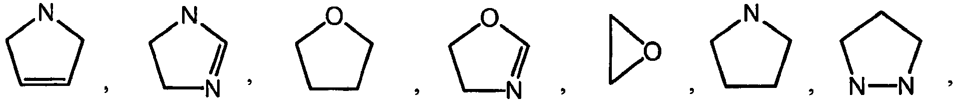 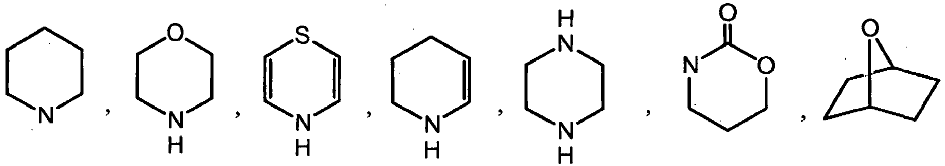 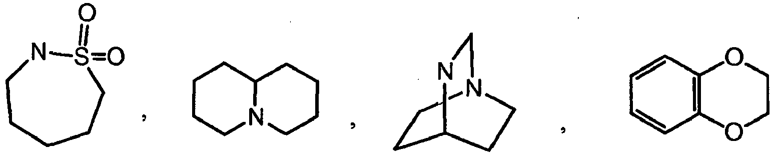 and the like. The term heteroalicyclic also includes all ring forms of the carbohydrates, including but not limited to the monosaccharides, the disaccharides and the oligosaccharides.

The term "moiety" refers to a specific segment or functional group of a molecule. Chemical moieties are often recognized chemical entities embedded in or appended to a molecule.

The term "bond" or "single bond" refers to a chemical bond between two atoms, or two moieties when the atoms joined by the bond are considered to be part of larger substructure.

The term "optionally substituted" means that the referenced group is optionally substituted with one or more additional group(s) individually and independently selected from alkyl, cycloalkyl, aryl, heteroaryl, heteroalicyclic, hydroxy, alkoxy, aryloxy, mercapto, alkylthio, arylthio, cyano, halo, carbonyl, thiocarbonyl, isocyanato, thiocyanato, isothiocyanato, nitro, perhaloalkyl, perfluoroalkyl, silyl, and amino, including mono- and di-substituted amino groups, and the protected derivatives thereof unless particularly specified.

The compounds presented herein may possess one or more chiral centers and each center may exist in the R or S configuration. The compounds presented herein include all diastereomeric, enantiomeric, and epimeric forms as well as the appropriate mixtures thereof. Stereoisomers may be obtained, if desired, for example, by the separation of stereoisomers by chiral chromatographic columns.

The methods and formulations described herein include the use of *N*-oxides, crystalline forms (also known as polymorphs), or pharmaceutically acceptable salts of compounds having the structure of Formula (II). By way of example only, a metabolite of fenretinide is *N*-(4-methoxyphenyl)retinamide, also known as 4-MPR or MPR. Another metabolite of fenretinide is 4-oxo fenretinide. In some situations, compounds may exist as tautomers. All tautomers are included within the scope of the compounds presented herein. In addition, the compounds described herein can exist in unsolvated as well as solvated forms with pharmaceutically acceptable solvents such as water, ethanol, and the like. The solvated forms of the compounds presented herein are also considered to be disclosed herein.

### PHARMACEUTICAL COMPOSITIONS

Another aspect are pharmaceutical compositions comprising a compound of Formula (II) and a pharmaceutically acceptable diluent, excipient, or carrier.

The term "pharmaceutical composition" refers to a mixture of a compound of Formula (II) with other chemical components, such as carriers, stabilizers, diluents, dispersing agents, suspending agents, thickening agents, and/or excipients. The pharmaceutical composition facilitates administration of the compound to an organism. Multiple techniques of administering a compound of Formula (II) include, but are not limited to: intravenous, oral, aerosol, parenteral, ophthalmic, pulmonary and topical administration.

The term "carrier" refers to relatively nontoxic chemical compounds or agents that facilitate the incorporation of a compound into cells or tissues.

The term "diluent" refers to chemical compounds that are used to dilute the compound of interest prior to delivery. Diluents are also optionally used to stabilize compounds. Salts dissolved in buffered solutions (which also provide pH control or maintenance) are optionally utilized as diluents, including, but not limited to a phosphate buffered saline solution.

The term "physiologically acceptable" refers to a material, such as a carrier or diluent, that does not abrogate the biological activity or properties of the compound, and is nontoxic.

The term "pharmaceutically acceptable salt" refers to a formulation of a compound that does not cause significant irritation to an organism to which it is administered and does not abrogate the biological activity and properties of the compound. Pharmaceutically acceptable salts are optionally obtained by reacting a compound of Formula (II) with acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutically acceptable salts are also optionally obtained by reacting a compound of Formula (II) with a base to form a salt such as an ammonium salt, an alkali metal salt, such as a sodium or a potassium salt, an alkaline earth metal salt, such as a calcium or a magnesium salt, a salt of organic bases such as dicyclohexylamine, *N*-methyl-D-glucamine, tris(hydroxymethyl)methylamine, and salts with amino acids such as arginine, lysine, and the like.

A "metabolite" of a compound disclosed herein is a derivative of that compound that is formed when the compound is metabolized. The term "active metabolite" refers to a biologically active derivative of a compound that is formed when the compound is metabolized. The term "metabolized" refers to the sum of the processes (including, but not limited to, hydrolysis reactions and reactions catalyzed by enzymes) by which a particular substance is changed by an organism. Thus, enzymes may produce specific structural alterations to a compound. For example, cytochrome P450 catalyzes a variety of oxidative and reductive reactions while uridine diphosphate glucuronyltransferases catalyze the transfer of an activated glucuronic-acid molecule to aromatic alcohols, aliphatic alcohols, carboxylic acids, amines and free sulphydryl groups. Further information on metabolism may be obtained from The Pharmacological Basis of Therapeutics, 9th Edition, McGraw-Hill (1996).

In some embodiments, metabolites of the compounds disclosed herein are identified either by administration of compounds to a host and analysis of tissue samples from the host, or by incubation of compounds with hepatic cells in vitro and analysis of the resulting compounds.

By way of example only, MPR is a metabolite of HPR, both of which are contained within the structure of Formula (II). MPR accumulates systemically in patients that have been chronically treated with HPR. One of the reasons that MPR accumulates systemically is that MPR is only (if at all) slowly metabolized, whereas HPR is metabolized to MPR. In addition, MPR may undergo relatively slow clearance. Thus, (a) the pharmacokinetics and pharmacodynamics of MPR must be taken into consideration when administering and determining the bioavailability of HPR, (b) MPR is more stable to metabolism than HPR, and (c) MPR can be more immediately bioavailable than HPR following absorption. Another metabolite of fenretinide is 4-oxo fenretinide.

MPR is also considered an active metabolite. MPR (like HPR) can bind to Retinol Binding Protein (RBP) and prevent the binding of RBP to Transerythrin (TTR). As a result, when either HPR or MPR is administered to a patient, one of the resulting expected features is that MPR will accumulate and bind to RBP and inhibit binding of retinol to RBP, as well as the binding of RBP to TTR. Accordingly, MPR can (a) serve as an inhibitor of retinol binding to RBP, (b) serve as an inhibitor of RBP to TTR, (c) limit the transport of retinol to certain tissues, including ophthalmic tissues, and (d) be transported by RBP to certain tissues, including ophthalmic tissues. MPR appears to bind more weakly to RBP than HPR, and is thus a less strong inhibitor of retinol binding to RBP. Nevertheless, both MPR and HPR are expected to inhibit, approximately equivalently, the binding of RBP to TTR. MPR has, in these respects, the same mode of action as HPR and can serve as a therapeutic agent in the methods and compositions described herein.

A "prodrug" refers to an agent that is converted into the parent drug *in vivo.* Prodrugs are often useful because, in some situations, they are easier to administer than the parent drug. Some prodrugs are, for instance, bioavailable by oral administration whereas the parent is not. Some prodrugs also have improved solubility in pharmaceutical compositions over the parent drug. An example, without limitation, of a prodrug is a compound of Formula (II) which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug is a short peptide (polyaminoacid) bonded to an acid group where the peptide is metabolized to reveal the active moiety.

In some embodiments, the compounds described herein are administered to a human patient *per se*, or in pharmaceutical compositions where they are mixed with other active ingredients, as in combination therapy, or suitable carrier(s) or excipient(s). Techniques for formulation and administration of the compounds of the instant application may be found in "Remington: The Science and Practice of Pharmacy," 20th ed. (2000).

### Routes Of Administration

Suitable routes of administration are, for example, oral, rectal, transmucosal, transdermal, pulmonary, or intestinal administration; parenteral delivery, including intramuscular, subcutaneous, intravenous, intramedullary injections, as well as intrathecal, direct intraventricular, intraperitoneal, or intranasal injections. In some embodiments, the compounds disclosed herein are administered orally.

### Composition/Formulation

Pharmaceutical compositions comprising a compound of Formula (II) are optionally manufactured in a manner that is itself known, *e.g*., by means of conventional mixing, dissolving, granulating, dragee-making, levigating, emulsifying, encapsulating, entrapping or compression processes.

Pharmaceutical compositions are optionally formulated in conventional manner using one or more physiologically acceptable carriers comprising excipients and auxiliaries which facilitate processing of the active compounds into preparations which can be used pharmaceutically. Proper formulation is dependent upon the route of administration chosen. The techniques, carriers, and excipients described in the art also suitable; *e.g.*, in Remington's Pharmaceutical Sciences.

The compounds of Formula (II) are optionally administered in a variety of ways, including systemically, such as orally or intravenously.

In some embodiments, a composition comprising a compound of Formula (I) illustratively take the form of a liquid where the agents are present in solution, in suspension or both. Typically when the composition is administered as a solution or suspension a first portion of the agent is present in solution and a second portion of the agent is present in particulate form, in suspension in a liquid matrix. In some embodiments, a liquid composition comprises a gel formulation. In other embodiments, the liquid composition is aqueous. In certain embodiments, the composition takes the form of an ointment.

Useful aqueous suspension can also contain one or more polymers as suspending agents. Useful polymers include water-soluble polymers such as cellulosic polymers, *e.g*., hydroxypropyl methylcellulose, and water-insoluble polymers such as cross-linked carboxyl-containing polymers. Useful compositions can also comprise an acceptable mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Useful compositions also include solubilizing agents to aid in the solubility of a compound of Formula (II). The term "solubilizing agent" generally includes agents that result in formation of a micellar solution or a true solution of the agent. Certain acceptable nonionic surfactants, for example polysorbate 80, can be useful as solubilizing agents, as can acceptable glycols, polyglycols, *e.g*., polyethylene glycol 400, and glycol ethers.

Useful compositions also include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Useful compositions also include one or more acceptable salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Other useful compositions also include one or more acceptable preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Still other useful compositions also include one or more acceptable surfactants to enhance physical stability or for other purposes. Suitable nonionic surfactants include polyoxyethylene fatty acid glycerides and vegetable oils, *e.g*., polyoxyethylene (60) hydrogenated castor oil; and polyoxyethylene alkylethers and alkylphenyl ethers, *e.g*., octoxynol 10, octoxynol 40.

Still other useful compositions include one or more antioxidants to enhance chemical stability where required. Suitable antioxidants include, by way of example only, ascorbic acid and sodium metabisulfite.

Aqueous suspension compositions can be packaged in single-dose non-reclosable containers. Alternatively, multiple-dose reclosable containers can be used, in which case it is typical to include a preservative in the composition.

One useful formulation for solubilizing higher quantities of the compounds of Formula (II) are, by way of example only, positively, negatively or neutrally charged phospholipids, or bile salt/phosphatidylcholine mixed lipid aggregate systems, such as those described in Li, C.Y., et al., Pharm. Res. 13:907-913 (1996). In some embodiments, an additional formulation that is used for the same purpose with compounds having the structure of Formula (II) involves use of a solvent comprising an alcohol, such as ethanol, in combination with an alkoxylated caster oil. *See, e.g.,* U.S. Patent Publication Number 2002/0183394. Or, alternatively, a formulation comprising a compound of Formula (II) is an emulsion composed of a lipoid dispersed in an aqueous phase, a stabilizing amount of a non-ionic surfactant, optionally a solvent, and optionally an isotonic agent. *See id.* Yet another formulation comprising a compound of Formula (II) includes corn oil and a non-ionic surfactant. *See* U.S. Patent No. 4,665,098. Still another formulation comprising a compound of Formula (II) includes lysophosphatidylcholine, monoglyceride and a fatty acid. *See* U.S. Patent No. 4,874,795. Still another formulation comprising a compound of Formula (II) includes flour, a sweetener, and a humectant. *See* International Publication No. WO 2004/069203. And still another formulation comprising a compound of Formula (II) includes dimyristoyl phosphatidylcholine, soybean oil, t-butyl alcohol and water. *See* U.S. Patent Application Publication No. US 2002/0143062.

For oral administration, compounds of Formula (II) are optionally formulated by combining the active compounds with art-recognized pharmaceutically acceptable carriers or excipients. Such carriers enable the compounds described herein to be formulated as tablets, powders, pills, dragees, capsules, liquids, gels, syrups, elixirs, slurries, suspensions and the like, for oral ingestion by a patient to be treated. In some embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. If desired, disintegrating agents may be added, such as the cross-linked croscarmellose sodium, polyvinylpyrrolidone, agar, or alginic acid or a salt thereof such as sodium alginate.

Dragee cores are provided with suitable coatings. For this purpose, in some embodiments, concentrated sugar solutions are used, which may optionally contain gum arabic, talc, polyvinylpyrrolidone, carbopol gel, polyethylene glycol, and/or titanium dioxide, lacquer solutions, and suitable organic solvents or solvent mixtures. In some embodiments, dyestuffs or pigments are added to the tablets or dragee coatings for identification or to characterize different combinations of active compound doses.

In some embodiments, pharmaceutical preparations which are used orally include push-fit capsules made of gelatin, including by way of example only, soft, sealed capsules made of gelatin and a plasticizer, such as glycerol or sorbitol; or hard-gel capsules or tablets. In certain embodiments, the push-fit capsules contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal or sublingual administration, in some embodiments, the compositions take the form of tablets, lozenges, or gels formulated in conventional manner.

In some embodiments, the active ingredient is in powder form for constitution with a suitable vehicle, *e.g*., sterile pyrogen-free water, before use.

A pharmaceutical carrier for the hydrophobic compounds of Formula (II) is a cosolvent system comprising benzyl alcohol, a nonpolar surfactant, a water-miscible organic polymer, and an aqueous phase. In some embodiments, the cosolvent system is a 10% ethanol, 10% polyethylene glycol 300, 10% polyethylene glycol 40 castor oil (PEG-40 castor oil) with 70% aqueous solution. This cosolvent system dissolves hydrophobic compounds well, and itself produces low toxicity upon systemic administration. Naturally, the proportions of a cosolvent system may be varied considerably without destroying its solubility and toxicity characteristics. Furthermore, the identity of the cosolvent components may be varied: for example, other low-toxicity nonpolar surfactants may be used instead of PEG-40 castor oil, the fraction size of polyethylene glycol 300 may be varied; other biocompatible polymers may replace polyethylene glycol, *e.g*., polyvinyl pyrrolidone; and other sugars or polysaccharides maybe included in the aqueous solution.

In some embodiments, other delivery systems for hydrophobic pharmaceutical compounds are employed. Liposomes and emulsions are examples of delivery vehicles or carriers for hydrophobic drugs. In some embodiments, organic solvents such as *N-*methylpyrrolidone are employed, although usually at the cost of greater toxicity. In other embodiments, the compounds are delivered using a sustained-release system, such as semipermeable matrices of solid hydrophobic polymers containing the therapeutic agent. In some embodiments, sustained-release capsules, depending on their chemical nature, release the compounds for a few weeks up to over 100 days. Depending on the chemical nature and the biological stability of the therapeutic reagent, additional strategies for protein stabilization may be employed.

One formulation for the administration of compounds having the structure of Formula (II) has been used with fenretinide in the treatment of neuroblastoma, prostate and ovarian cancers, and is marketed by Avanti Polar Lipids, Inc. (Alabaster, Alabama) under the name Lym-X-Sorb™. This formulation, which comprises an organized lipid matrix that includes lysophosphatidylcholine, monoglyceride and fatty acid, is designed to improve the oral availability of fenretinide. Such a formulation, *i.e.*, an oral formulation that includes lysophosphatidylcholine, monoglyceride and fatty acid, is proposed to also provide improved bioavailability of compounds having the structure of Formula (II) for the treatment of ophthalmic and ocular diseases and conditions, including but not limited to the macular degenerations and dystrophies. In some embodiments, this formulation is used in a range of orally-administered compositions, including by way of example only, a capsule and a powder that is suspended in water to form a drinkable composition.

In some embodiments, all of the formulations described herein benefit from antioxidants, metal chelating agents, thiol containing compounds and other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (I) pentosan polysulfate and other heparinoids, (m) divalent cations such as magnesium and zinc; or (n) combinations thereof.

In some embodiments, many of the compounds of Formula (II) are provided as salts with pharmaceutically compatible counterions. In one embodiment, pharmaceutically compatible salts are formed with many acids, including but not limited to hydrochloric, sulfuric, acetic, lactic, tartaric, malic, succinic, *etc.* Salts tend to be more soluble in aqueous or other protonic solvents than are the corresponding free acid or base forms.

### TREATMENT METHODS, DOSAGES AND COMBINATION THERAPIES

The term "mammal" means all mammals including humans. Mammals include, by way of example only, humans, non-human primates, cows, dogs, cats, goats, sheep, pigs, rats, mice and rabbits.

The term "effective amount" as used herein refers to that amount of the compound being administered which will relieve to some extent one or more of the symptoms of the disease, condition or disorder being treated.

In some embodiments, the compositions containing the compound(s) described herein are administered for prophylactic and/or therapeutic treatments. The term "treating" is used to refer to either prophylactic and/or therapeutic treatments. In therapeutic applications, the compositions are administered to a patient already suffering from a disease, condition or disorder, in an amount sufficient to cure or at least partially arrest the symptoms of the disease, disorder or condition. Amounts effective for this use will depend on the severity and course of the disease, disorder or condition, previous therapy, the patient's health status and response to the drugs, and the judgment of the treating physician.

In prophylactic applications, compositions containing the compounds described herein are administered to a patient susceptible to or otherwise at risk of a particular disease, disorder or condition. Such an amount is defined to be a "prophylactically effective amount or dose." In this use, the precise amounts also depend on the patient's state of health, weight, and the like.

The terms "enhance" or "enhancing" means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system. When used in a patient, amounts effective for this use will depend on the severity and course of the disease, disorder or condition, previous therapy, the patient's health status and response to the drugs, and the judgment of the treating physician.

In the case wherein the patient's condition does not improve, in some embodiments, upon the doctor's discretion the administration of the compounds are administered chronically, that is, for an extended period of time, including throughout the duration of the patient's life in order to ameliorate or otherwise control or limit the symptoms of the patient's disease or condition.

In the case wherein the patient's status does improve, in some embodiments, upon the doctor's discretion the administration of the compounds are given continuously or temporarily suspended for a certain length of time (*i.e.*, a "drug holiday").

Once improvement of the patient's conditions has occurred, a maintenance dose is administered if necessary. Subsequently, the dosage or the frequency of administration, or both, can be reduced, as a function of the symptoms, to a level at which the improved disease, disorder or condition is retained. Patients can, however, require intermittent treatment on a long-term basis upon any recurrence of symptoms.

The amount of a given agent that will correspond to such an amount will vary depending upon factors such as the particular compound, disease condition and its severity, the identity (*e.g*., weight) of the subject or host in need of treatment, but can nevertheless be determined in a manner according to the particular circumstances surrounding the case, including, *e.g*., the specific agent being administered, the route of administration, the condition being treated, and the subject or host being treated. In general, however, doses employed for adult human treatment will typically be in the range of 0.02-5000 mg per day, preferably 1-1500 mg per day. In some embodiments, doses employed for adult human treatment will typically be in the range of 50-500 mg per day. In some embodiments, doses employed for adult human treatment will about 100 mg per day, about 200 mg per day, about 300 mg per day, about 400 mg per day, or about 500 mg per day. In some embodiments, the desired dose is conveniently presented in a single dose or as divided doses administered simultaneously (or over a short period of time) or at appropriate intervals, for example as two, three, four or more sub-doses per day.

In certain instances, it is appropriate to administer at least one of the compounds described herein (or a pharmaceutically acceptable salt, ester, amide, prodrug, or solvate) in combination with another therapeutic agent. By way of example only, in some embodiments, if one of the side effects experienced by a patient upon receiving one of the compounds herein is inflammation, then it is appropriate to administer an anti-inflammatory agent in combination with the initial therapeutic agent. Or, by way of example only, in some embodiments, the therapeutic effectiveness of one of the compounds described herein is enhanced by administration of an adjuvant (*i.e.*, by itself the adjuvant may only have minimal therapeutic benefit, but in combination with another therapeutic agent, the overall therapeutic benefit to the patient is enhanced). Or, by way of example only, in some embodiments, the benefit of experienced by a patient is increased by administering one of the compounds described herein with another therapeutic agent (which also includes a therapeutic regimen) that also has therapeutic benefit. By way of example only, in some embodiments, in a treatment for macular degeneration involving administration of one of the compounds described herein, increased therapeutic benefit result by also providing the patient with other therapeutic agents or therapies for macular degeneration. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of at least one compound of formula (II) with nitric oxide (NO) inducers, statins, negatively charged phospholipids, anti-oxidants, minerals, anti-inflammatory agents, anti-angiogenic agents, matrix metalloproteinase inhibitors, and carotenoids. In certain embodiments, in several instances, suitable combination agents fall within multiple categories (by way of example only, lutein is an anti-oxidant and a carotenoid). Further, in certain embodiments, the compounds of Formula (II) are also administered with additional agents that provide benefit to the patient, including by way of example only cyclosporin A.

In addition, in some embodiments, the compounds of Formula (II) is also used in combination with procedures that provide additional or synergistic benefit to the patient, including, by way of example only, the use of extracorporeal rheopheresis (also known as membrane differential filtration), the use of implantable miniature telescopes, laser photocoagulation of drusen, and microstimulation therapy.

The use of anti-oxidants has been shown to benefit patients with macular degenerations and dystrophies. *See, e.g.,* Arch. Ophthalmol., 119: 1417-36 (2001); Sparrow, et al., J. Biol. Chem., 278:18207-13 (2003). Examples of suitable anti-oxidants that could be used in combination with at least one compound having the structure of Formula (II) include vitamin C, vitamin E, beta-carotene and other carotenoids, coenzyme Q, 4-hydroxy-2,2,6,6-tetramethylpiperidine-*N*-oxyl (also known as Tempol), lutein, butylated hydroxytoluene, resveratrol, a trolox analogue (PNU-83836-E), and bilberry extract.

The use of certain minerals has also been shown to benefit patients with macular degenerations and dystrophies. *See, e.g.,* Arch. Ophthalmol., 119: 1417-36 (2001). Examples of suitable minerals that could be used in combination with at least one compound having the structure of Formula (II) include copper-containing minerals, such as cupric oxide (by way of example only); zinc-containing minerals, such as zinc oxide (by way of example only); and selenium-containing compounds.

The use of certain negatively-charged phospholipids has also been shown to benefit patients with macular degenerations and dystrophies. *See, e.g.*, Shaban & Richter, Biol. Chem., 383:537-45 (2002); Shaban, et al., Exp. Eye Res., 75:99-108 (2002). Examples of suitable negatively charged phospholipids that could be used in combination with at least one compound having the structure of Formula (II) include cardiolipin and phosphatidylglycerol. In certain embodiments, positively-charged and/or neutral phospholipids also provide benefit for patients with macular degenerations and dystrophies when used in combination with compounds having the structure of Formula (II).

The use of certain carotenoids has been correlated with the maintenance of photoprotection necessary in photoreceptor cells. Carotenoids are naturally-occurring yellow to red pigments of the terpenoid group that can be found in plants, algae, bacteria, and certain animals, such as birds and shellfish. Carotenoids are a large class of molecules in which more than 600 naturally occurring carotenoids have been identified. Carotenoids include hydrocarbons (carotenes) and their oxygenated, alcoholic derivatives (xanthophylls). They include actinioerythrol, astaxanthin, canthaxanthin, capsanthin, capsorubin, β-8'-apo-carotenal (apo-carotenal), β-12'-apo-carotenal, α-carotene, β-carotene, "carotene" (a mixture of α- and β-carotenes), γ-carotenes, β -cyrptoxanthin, lutein, lycopene, violerythrin, zeaxanthin, and esters of hydroxyl- or carboxyl-containing members thereof. Many of the carotenoids occur in nature as *cis-* and trans-isomeric forms, while synthetic compounds are frequently racemic mixtures.

In humans, the retina selectively accumulates mainly two carotenoids: zeaxanthin and lutein. These two carotenoids are thought to aid in protecting the retina because they are powerful antioxidants and absorb blue light. Studies with quails establish that groups raised on carotenoid-deficient diets had retinas with low concentrations of zeaxanthin and suffered severe light damage, as evidenced by a very high number of apoptotic photoreceptor cells, while the group with high zeaxanthin concentrations had minimal damage. Examples of suitable carotenoids for in combination with at least one compound having the structure of Formula (II) includes lutein and zeaxanthin, as well as any of the aforementioned carotenoids.

Suitable nitric oxide inducers include compounds that stimulate endogenous NO or elevate levels of endogenous endothelium-derived relaxing factor (EDRF) in vivo or are substrates for nitric oxide synthase. Such compounds include, for example, L-arginine, L-homoarginine, and *N*-hydroxy-*L*-arginine, including their nitrosated and nitrosylated analogs (*e.g.*, nitrosated L-arginine, nitrosylated L-arginine, nitrosated *N*-hydroxy-*L-*arginine, nitrosylated *N*-hydroxy-*L*-arginine, nitrosated *L*-homoarginine and nitrosylated *L-*homoarginine), precursors of *L*-arginine and/or physiologically acceptable salts thereof, including, for example, citrulline, ornithine, glutamine, lysine, polypeptides comprising at least one of these amino acids, inhibitors of the enzyme arginase (*e.g.*, *N*-hydroxy-*L-*arginine and 2(S)-amino-6-boronohexanoic acid) and the substrates for nitric oxide synthase, cytokines, adenosine, bradykinin, calreticulin, bisacodyl, and phenolphthalein. EDRF is a vascular relaxing factor secreted by the endothelium, and has been identified as nitric oxide or a closely related derivative thereof (Palmer et al, Nature, 327:524-526 (1987); Ignarro et al, Proc. Natl. Acad. Sci. USA, 84:9265-9269 (1987)).

Statins serve as lipid-lowering agents and/or suitable nitric oxide inducers. In addition, a relationship has been demonstrated between statin use and delayed onset or development of macular degeneration. G. McGwin, et al., British Journal of Ophthalmology, 87:1121-25 (2003). Statins can thus provide benefit to a patient suffering from an ophthalmic condition (such as the macular degenerations and dystrophies, and the retinal dystrophies) when administered in combination with compounds of Formula (II). Suitable statins include, by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin.

In some embodiments, suitable anti-inflammatory agents with which the Compounds of Formula (II) are used include, by way of example only, aspirin and other salicylates, cromolyn, nedocromil, theophylline, zileuton, zafirlukast, montelukast, pranlukast, indomethacin, and lipoxygenase inhibitors; non-steroidal antiinflammatory drugs (NSAIDs) (such as ibuprofen and naproxin); prednisone, dexamethasone, cyclooxygenase inhibitors (*i.e.*, COX-1 and/or COX-2 inhibitors such as Naproxen™, or Celebrex™); statins (by way of example only, rosuvastatin, pitivastatin, simvastatin, pravastatin, cerivastatin, mevastatin, velostatin, fluvastatin, compactin, lovastatin, dalvastatin, fluindostatin, atorvastatin, atorvastatin calcium (which is the hemicalcium salt of atorvastatin), and dihydrocompactin); and disassociated steroids.

In other embodiments, suitable matrix metalloproteinases (MMPs) inhibitors are also administered in combination with compounds of Formula (II) in order to treat ophthalmic conditions or symptoms associated with macular or retinal degenerations. MMPs are known to hydrolyze most components of the extracellular matrix. These proteinases play a central role in many biological processes such as normal tissue remodeling, embryogenesis, wound healing and angiogenesis. However, excessive expression of MMP has been observed in many disease states, including macular degeneration. Many MMPs have been identified, most of which are multidomain zinc endopeptidases. A number of metalloproteinase inhibitors are known (see for example the review of MMP inhibitors by Whittaker M. et al, Chemical Reviews 99(9):2735-2776 (1999)). Representative examples of MMP Inhibitors include Tissue Inhibitors of Metalloproteinases (TIMPs) (*e.g*., TIMP-1, TIMP-2, TIMP-3, or TIMP-4), α₂-macroglobulin, tetracyclines (*e.g*., tetracycline, minocycline, and doxycycline), hydroxamates (*e.g.*, BATIMASTAT, MARIMISTAT and TROCADE), chelators (*e.g.*, EDTA, cysteine, acetylcysteine, D-penicillamine, and gold salts), synthetic MMP fragments, succinyl mercaptopurines, phosphonamidates, and hydroxaminic acids. Examples of MMP inhibitors that are used in combination with compounds of Formula (II) include, by way of example only, any of the aforementioned inhibitors.

The use of antiangiogenic or anti-VEGF drugs has also been shown to provide benefit for patients with macular degenerations and dystrophies. Examples of suitable antiangiogenic or anti-VEGF drugs that could be used in combination with at least one compound having the structure of Formula (II) include Rhufab V2 (Lucentis™), Tryptophanyl-tRNA synthetase (TrpRS), Eye001 (Anti-VEGF Pegylated Aptamer), squalamine, Retaane™ 15mg (anecortave acetate for depot suspension; Alcon, Inc.), Combretastatin A4 Prodrug (CA4P), Macugen™, Mifeprex™ (mifepristone - ru486), subtenon triamcinolone acetonide, intravitreal crystalline triamcinolone acetonide, Prinomastat (AG3340 - synthetic matrix metalloproteinase inhibitor, Pfizer), fluocinolone acetonide (including fluocinolone intraocular implant, Bausch & Lomb/Control Delivery Systems), VEGFR inhibitors (Sugen), and VEGF-Trap (Regeneron/Aventis). Resveratrol, which can be extracted from walnuts or the skins of red grapes, has demonstrated anti-angiogenic activity and in some embodiments, is used as the second or additional agent for the combination therapies described herein. Furthermore, other trans-stilbene compounds are expected to exhibit similar activity.

Other pharmaceutical therapies that have been used to relieve visual impairment are optionally used in combination with at least one compound of Formula (II). Such treatments include but are not limited to agents such as Visudyne™ with use of a non-thermal laser, PKC 412, Endovion (NeuroSearch A/S), neurotrophic factors, including by way of example Glial Derived Neurotrophic Factor and Ciliary Neurotrophic Factor, diatazem, dorzolamide, Phototrop, 9-cis-retinal, eye medication (including Echo Therapy) including phospholine iodide or echothiophate or carbonic anhydrase inhibitors, AE-941 (AEterna Laboratories, Inc.), Sirna-027 (Sirna Therapeutics, Inc.), pegaptanib (NeXstar Pharmaceuticals/Gilead Sciences), neurotrophins (including, by way of example only, NT-4/5, Genentech), Cand5 (Acuity Pharmaceuticals), ranibizumab (Genentech), INS-37217 (Inspire Pharmaceuticals), integrin antagonists (including those from Jerini AG and Abbott Laboratories), EG-3306 (Ark Therapeutics Ltd.), BDM-E (BioDiem Ltd.), thalidomide (as used, for example, by EntreMed, Inc.), cardiotrophin-1 (Genentech), 2-methoxyestradiol (Allergan/Oculex), DL-8234 (Toray Industries), NTC-200 (Neurotech), tetrathiomolybdate (University of Michigan), LYN-002 (Lynkeus Biotech), microalgal compound (Aquasearch/Albany, Mera Pharmaceuticals), D-9120 (Celltech Group pic), ATX-S10 (Hamamatsu Photonics), TGF-beta 2 (Genzyme/Celtrix), tyrosine kinase inhibitors (Allergan, SUGEN, Pfizer), NX-278-L (NeXstar Pharmaceuticals/Gilead Sciences), Opt-24 (OPTIS France SA), retinal cell ganglion neuroprotectants (Cogent Neurosciences), N-nitropyrazole derivatives (Texas A&M University System), KP-102 (Krenitsky Pharmaceuticals), and cyclosporin A. *See* U.S. Patent Application Publication No. 20040092435.

In any case, in some embodiments, the multiple therapeutic agents (one of which is one of the compounds described herein) are administered in any order or even simultaneously. In certain embodiments, if simultaneously, the multiple therapeutic agents are provided in a single, unified form, or in multiple forms (by way of example only, either as a single pill or as two separate pills). In some embodiments, one of the therapeutic agents is given in multiple doses, or both are given as multiple doses. If not simultaneous, in some embodiments, the timing between the multiple doses vary from more than zero weeks to less than four weeks. In addition, the combination methods, compositions and formulations are not to be limited to the use of only two agents; the use of multiple therapeutic combinations was envisioned. By way of example only, in some embodiments, a compound having the structure of Formula (II) is provided with at least one antioxidant and at least one negatively charged phospholipid; or a compound having the structure of Formula (II) is provided with at least one antioxidant and at least one inducer of nitric oxide production; or a compound having the structure of Formula (II) is provided with at least one inducer of nitric oxide productions and at least one negatively charged phospholipid; and so forth.

In some embodiments, the compounds of Formula (II) are also used in combination with procedures that provide additional or synergistic benefit to the patient. Procedures known, proposed or considered to relieve visual impairment include but are not limited to 'limited retinal translocation', photodynamic therapy (including, by way of example only, receptor-targeted PDT, Bristol-Myers Squibb, Co.; porfimer sodium for injection with PDT; verteporfin, QLT Inc.; rostaporfin with PDT, Miravent Medical Technologies; talaporfin sodium with PDT, Nippon Petroleum; motexafin lutetium, Pharmacyclics" Inc.), antisense oligonucleotides (including, by way of example, products tested by Novagali Parma SA and ISIS-13650, Isis Pharmaceuticals), laser photocoagulation, drusen lasering, macular hole surgery, macular translocation surgery, implantable miniature telescopes, Phi-Motion Angiography (also known as Micro-Laser Therapy and Feeder Vessel Treatment), Proton Beam Therapy, microstimulation therapy, Retinal Detachment and Vitreous Surgery, Scleral Buckle, Submacular Surgery, Transpupillary Thermotherapy, Photosystem I therapy, use of RNA interference (RNAi), extracorporeal rheopheresis (also known as membrane differential filtration and Rheotherapy), microchip implantation, stem cell therapy, gene replacement therapy, ribozyme gene therapy (including gene therapy for hypoxia response element, Oxford Biomedica; Lentipak, Genetix; PDEF gene therapy, GenVec), photoreceptor/retinal cells transplantation (including transplantable retinal epithelial cells, Diacrin, Inc.; retinal cell transplant, Cell Genesys, Inc.), and acupuncture.

In other embodiments, further combinations that are used to benefit an individual include using genetic testing to determine whether that individual is a carrier of a mutant gene that is correlated with certain ophthalmic conditions. By way of example only, defects in the human *ABCA4* gene are thought to be associated with five distinct retinal phenotypes including Stargardt disease, cone-rod dystrophy, age-related macular degeneration and retinitis pigmentosa. *See e.g.*, Allikmets et al., Science, 277:1805-07 (1997); Lewis et al., Am. J. Hum. Genet., 64:422-34 (1999); Stone et al., Nature Genetics, 20:328-29 (1998); Allikmets, Am. J. Hum. Gen., 67:793-799 (2000); Klevering, et al, Ophthalmology, 111 :546-553 (2004). In addition, an autosomal dominant form of Stargardt Disease is caused by mutations in the *ELOV4* gene. *See* Karan, et al., Proc. Natl. Acad. Sci. (2005). Patients possessing any of these mutations are expected to find therapeutic and/or prophylactic benefit in the methods described herein.

In some embodiments, compounds of Formula (II) or other agents that result in the reduction of serum retinol levels are optionally administered with (meaning before, during or after) agents that treat or alleviate side effects arising from serum retinol reduction. Such side effects include dry skin and dry eye. Accordingly, agents that alleviate or treat either dry skin or dry eye are administered with compounds of Formula (II) or other agents that reduce serum retinol levels.

### ILLUSTRATIVE EXAMPLES

The following examples provide illustrative methods for testing the effectiveness and safety of the compounds of Formula (II).

**Example 1.** Spontaneously arising diabetes in the *Ins2Akita*/*+* mouse is due to a point mutation which disrupts proper folding of the mature insulin protein. This mutation leads to hyperglycemia and hypoinsulinemia in heterozygous mice by 4 weeks. In addition to increased retinal vascular permeability and an increase in acellular capillaries, *Ins2Akital+* mice demonstrate thinning of the inner plexiform and inner nuclear layers, and decrease in the number of cell bodies in the retinal ganglion cell layer (GCL). The presence of active caspase-3 in the GCL after 4 weeks of hyperglycemia is consistent with cell death by apoptosis.

Analyses of *Ins2Akital+* mice have revealed oxidative stress biomarkers (hydroxynonenal and nitrotyrosine) and elevated levels of permeability-mediating factors (p38 MAPK and VEGF). The documented retinal pathology and presence of angiogenic factors renders the *Ins2Akital+* mice as an appropriate model to examine the anti-angiogenic properties of HPR.

HPR is a retinoic acid derivative which mediates apoptotic cell death in oncogenic and transformed cell lines. Investigations of angiogenic properties of HPR in models of "natural" pathophysiology have not been previously reported. In this example, studies were designed to evaluate the effects of HPR on retinal pathology in the *Ins2Akita*/*+* diabetic mouse.

*Ins2Akita* littermates (aged 2 - 5 months) were divided into two groups. One group received a specialized rodent diet containing HPR (0.1%, w/w). The second group received a rodent chow which was not supplemented with HPR. Mice ingested these diets *ad libitum* for 3 months (except where indicated). Serum retinol and glucose levels were regularly monitored throughout the treatment period. At the end of the treatment period, the mice were euthanized and eyecups were prepared for biochemical and immunohistochemical analyses.

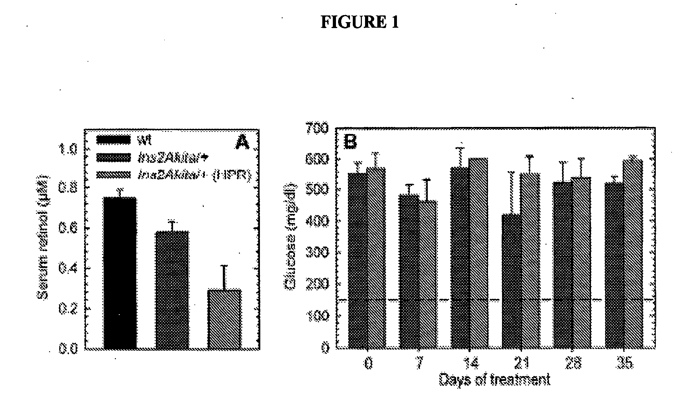

As shown in Figure 1, *Ins2Akital+* mice were fed either a control or HPR-supplemented diet as described in the Methods. Serum levels of retinol at day 30 (panel A) and glucose at 7-day intervals (panel B) are shown. The dashed line in panel B indicates mean glucose levels in wild-type mice.

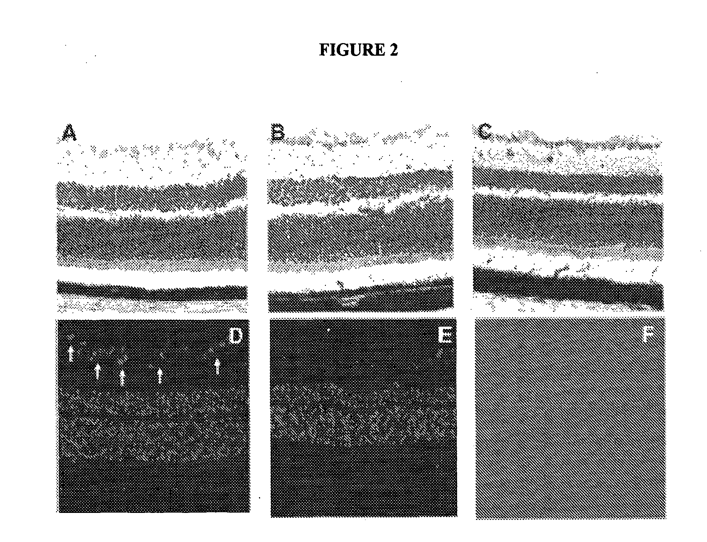

As shown in Figure 2, tissue sections above show toludine blue staining (panels A-C) and RAGE immunoreactivity (panels D-F). Panels A and D are from *Ins2Akita*/*+* mice fed the control diet. Panels B and E are from *Ins2*/*Akita*/*+* mice fed the HPR-supplemented diet. Panels C and E are from age-matched wild-type mice. Arrows in panels A and D show disruption of the GCL and RAGE immunoreactivity. Meanwhile, the GCL in HPR-treated mice is well preserved and shows very little RAGE immunoreactivity.

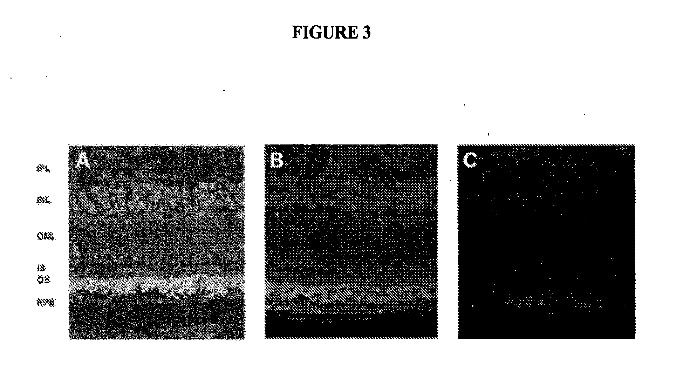

As shown in Figure 3, tissue sections from *Ins2Akita*/*+* mice fed either the control diet (panel A) or the HPR-supplemented diet (panel B), and age-matched wild-type mice (panel C), were probed for AGE immunoreactivity. *Ins2Akita*/*+* mice fed the control diet showed massive accumulation of AGEs throughout the retina. AGE immunoreactivity is significantly reduced in HPR-treated mice.

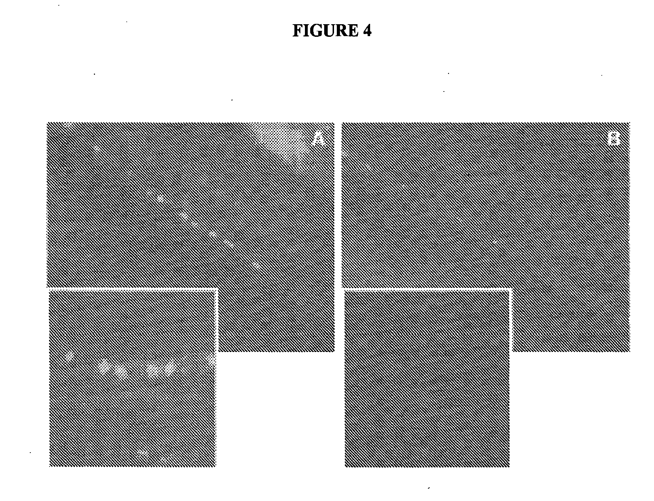

As shown in Figure 4, *Ins2Akita*/*+* mice were fed either the control diet (panel A) or the HPR-supplemented diet (panel B). Tissue sections from these mice were probed for phosphorylated p38 MAPK, a mediator of apoptotic cell death. *Ins2Akita*/*+* mice fed the control diet showed pronounced immunoreactivity within RPE cell nuclei (see high magnification confocal image inset in panel A). In marked contrast, p38 MAPK immunoreactivity was barely detectable in the RPE of HPR-treated mice (panel B and inset).

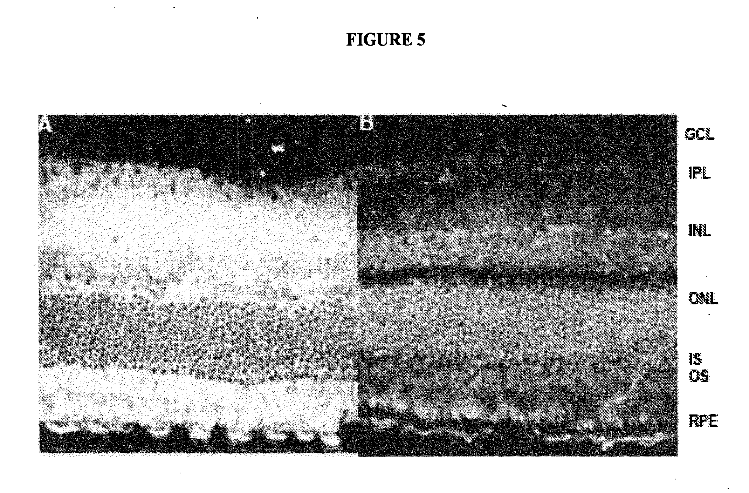

As shown in Figure 5, *Ins2Akital+* mice fed the control diet show diffuse and widespread VEGF expression in the retina (panel A). Meanwhile, *Ins2Akita*/*+* mice fed the HPR-supplemented diet show dramatically reduced VEGF expression in all retina sublayers (panel B). Notably, the ganglion cell layer and inner nuclear layer layers in HPR-treated mice show remarkable preservation.

From this study, we conclude that (a) HPR significantly reduces serum RBP-retinol but has no effect on hyperglycemia in the *Ins2Akita*/*+* diabetic mouse; (b) HPR treatment reduces expression of AGEs/RAGEs in the retina and preserves integrity of the ganglion cell layer; and (c) HPR treatment potently reduces p38 MAPK-mediated cell death in the RPE and downregulates VEGF expression.

**Example 2.** We set out to directly test the angiogenic properties of HPR in three different models of ocular angiogenesis. In the first, we induced angiogenesis *in vitro* by exposing primary human retinal microvascular endothelial cells to growth factors in the tube formation assay. Second, we utilized slow release pellets containing growth factor to induce angiogenesis in the corneal micropocket assay. Next, we looked at the effect of HPR treatment on angiogenesis in a transgenic animal model of early-onset retinal neovascularization, the very-low-density lipoprotein receptor (VLDLR) knockout mouse *(vldlr* -/-). We have found that, in three disparate models of ocular angiogenesis, HPR treatment resulted in a potent reduction in angiogenesis.

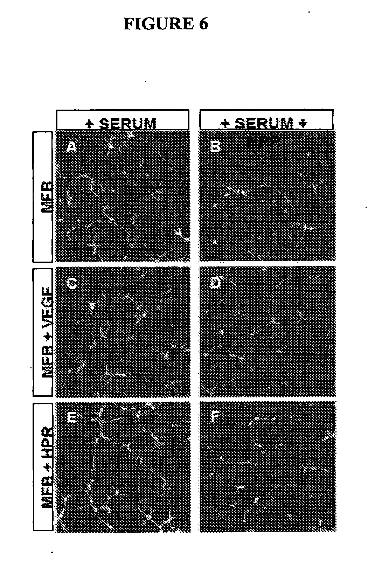

As shown in Figure 6, human retinal microvascular endothelial cells were placed in reduced serum media (MFB: 0.5% FBS, 0.1% BSA in MCDB131 medium) overnight prior to seeding on CultrexTM basement membrane extract (BME). In parallel experiments, cells were pretreated with VEGF (10ng/ml and HPR (10mM) respectively. The cells were then plated in 0.1% FBS or 0.1%FBS-10mM HPR. **Figure 6****:** Tube formation was reduced in the presence of HPR (B, D, F), indicating that HPR ameliorates growth-factor mediated angiogenesis.

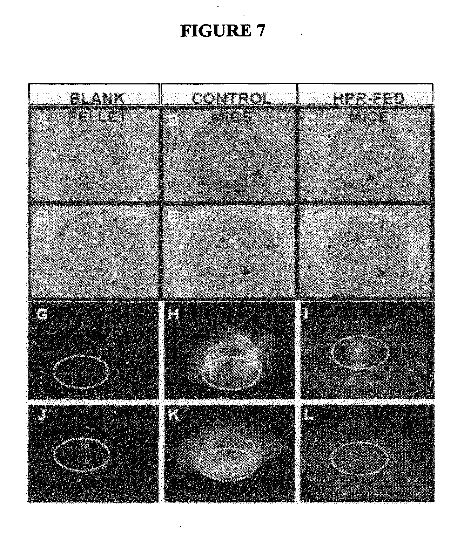

As shown in Figure 7, wild type Balb/c mice were fed either control or 0.1% (w/w) HPR-supplemented chow ad libitum for 8 weeks. At the end of this period, standardized slow release pellets containing bFGF (~ 80ng/pellet) were surgically inserted into the normally avascular corneas of the mice in the study. Blank pellets were implanted in the left eye. Vessel formation was assessed 5 days later when the mice were perfused with FITC-dextran and the eyes were enucleated and photographed. **Figure 7****:** Bright field (A-F) and fluorescence (G-L) images showing vessel growth (arrows) and the approximate location of the pellets (ovals). A strong angiogenic response was observed in mice fed the control diet (arrows, B,E,H,K). However, a striking reduction was observed (C,F,I,L) in HPR treated mice indicating that HPR potently inhibits the pro-angiogenic effects of bFGF.

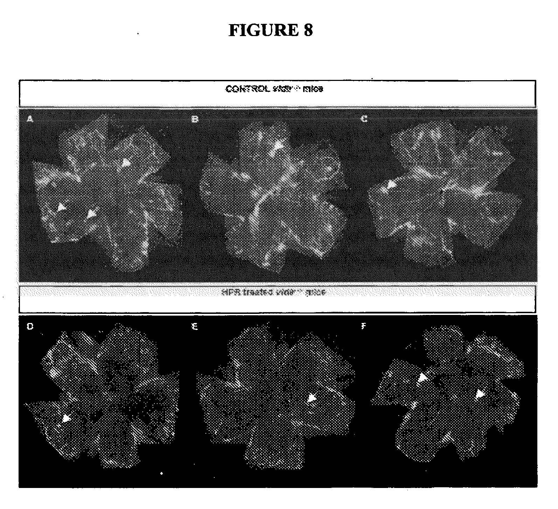

As shown in Figure 8, the VLDLR ^{-/-} knockout mice exhibit profound subretinal neovascularization which manifests as vascular leakage in the retina. VLDLR ^{-/-} mice were fed either control or 0.1% HPR (w/w)-supplemented chow ad libitum for 8 weeks. The extent of retinal leakage was then assessed in retinal flat mounts after FITC-dextran perfusion. **Figure 8****:** HPR treatment led to a significant reduction in the extent of retinal vascular leakage (arrows, D,E,F) in the *vldlr* ^{-/-} mice.

Findings from the present study clearly demonstrate that HPR does not augment or exacerbate growth factor-mediated retinal pathology. HPR potently inhibits growth factor-induced neovascularization and appears to ameliorate vascular leakage in a mouse model of retinal angiogenesis. Hence, we conclude that HPR has a predominantly anti-angiogenic, or at minimum, an angiostatic effect in each of the models tested.

All of the methods disclosed and claimed herein can be made and executed without undue experimentation in light of the present disclosure. Variations may be applied to the methods and in the steps or in the sequence of steps of the method described herein without departing from the concept, spirit and scope of the invention. More specifically, it will be apparent that certain agents that are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

## Claims

1. A compound of Formula (II) for use in the treatment of glaucoma, ocular hypertension or a combination thereof; in a human, wherein the compound of Formula (II) has the structure: 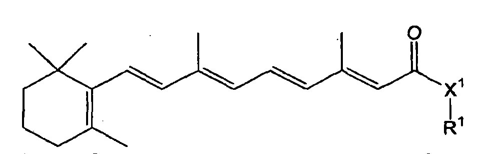 wherein X¹ is selected from the group consisting of NR², O, S, CHR²; R¹ is (CHR²)ₓ-L¹-R³, wherein x is 0, 1, 2, or 3; L¹ is a single bond or-C(O)-; R² is a moiety selected from the group consisting of H, (C₁-C₄)alkyl, F, (C₁-C₄)fluoroalkyl, (C₁-C₄)alkoxy, -C(O)OH,
-C(O)-NH₂, -(C₁-C₄)alkylamine, -C(O)-(C₁-C₄)alkyl, -C(O)-(C₁-C₄)fluoroalkyl, -C(O)-(C₁-C₄)alkylamine, and -C(O)-(C₁-C₄)alkoxy; and R³ is H or a moiety selected from the group consisting of (C₂-C₇)alkenyl, (C₂-C₇)alkynyl, aryl, (C₃-C₇)cycloalkyl, (C₅-C₇)cycloalkenyl, and a heterocycle, optionally substituted with 1-3 independently selected substituents; provided that R³ is not H when both x is 0 and L¹ is a single bond; or a pharmaceutically acceptable salt or solvate thereof.

2. A compound for use as claimed in claim 1, wherein the compound is 4-methoxyphenylretinamide.

3. A compound for use as claimed in claim 1, wherein x is 0.

4. A compound for use as claimed in claim 3, wherein R³ is an optionally substituted aryl.

5. A compound for use as claimed in claim 4, wherein X¹ is NH.

6. A compound for use as claimed in claim 5, wherein the aryl group has one substituent.

7. A compound for use as claimed in claim 6, wherein the substituent is a moiety selected from the group consisting of halogen, OH, O(C₁-C₄)alkyl, NH(C₁-C₄)alkyl, O(C₁-C₄)fluoroalkyl, and N[(C₁-C₄)alkyl]₂.

8. A compound for use as claimed in claim 7, wherein the substituent is OH.

9. A compound for use as claimed in claim 1, wherein the compound is 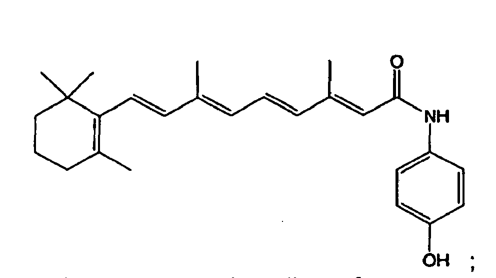 or a pharmaceutically acceptable solvate thereof.

10. A compound for use as claimed in claim 1, wherein the compound is 4-hydroxyphenylretinamide; or a pharmaceutically acceptable salt or solvate thereof.

11. A compound for use as claimed in claim 1, wherein the effective amount of the compound is systemically administered to the human.

12. A compound for use as claimed in claim 11, wherein the effective amount of the compound is administered orally to the human, for example as multiple administrations of the effective amount of the compound.

13. A compound for use as claimed in claim 1, further comprising administering at least one additional agent selected from the group consisting of an inducer of nitric oxide production, an anti-inflammatory agent, a physiologically acceptable antioxidant, a physiologically acceptable mineral, a negatively charged phospholipid, a carotenoid, a statin, an anti-angiogenic drug, a matrix metalloproteinase inhibitor, resveratrol and other trans-stilbene compounds, and 13-cis-retinoic acid.

14. A compound for use as claimed in claim 1, which is 4-oxo fenretinide.

## Patentansprüche

1. Verbindung der Formel (II) zum Gebrauch bei der Behandlung von grünem Star, Augeninnenhochdruck oder einer Kombination von diesen in einem Menschen, wobei die Verbindung der Formel (II) die folgende Struktur aufweist: 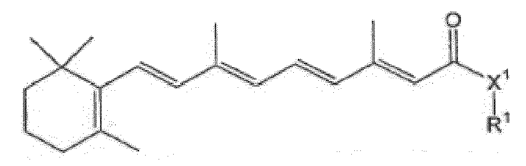 wobei X¹ ausgewählt ist aus der Gruppe bestehend aus NR², O, S, CHR²; R¹ (CHR²)ₓ-L¹-R³ ist, wobei x 0, 1, 2 oder 3 ist; L¹ eine Einfachbindung oder -C(O)- ist; R² ein Anteil, ausgewählt aus der Gruppe bestehend aus H, (C₁-C₄)alkyl, F, (C₁-C₄)fluoralkyl, (C₁-C₄)alkoxy, -C(O)OH, -C(O)-NH₂, -(C₁-C₄)alkylamin, -C(O)-(C₁-C₄)alkyl, -C(O)-(C₁-C₄)fluoralkyl, -C(O)-(C₁-C₄)alkylamin und -C(O)-(C₁-C₄)alkoxy, ist; und R³ H oder ein Anteil, ausgewählt aus der Gruppe bestehend aus (C₂-C₇)alkenyl, (C₂-C₇)alkinyl, Aryl, (C₃-C₇)cycloalkyl, (C₅-C₇)cycloalkenyl und einem Heterocyclus, ist, optional substituiert mit 1 - 3 unabhängig ausgewählten Substituenten; sofern R³ nicht H ist, wenn sowohl x 0 ist als auch L¹ eine Einfachbindung ist oder ein pharmazeutisch unbedenkliches Salz oder Solvat davon.

2. Verbindung zum Gebrauch nach Anspruch 1, wobei die Verbindung 4-Methoxyphenylretinamid ist.

3. Verbindung zum Gebrauch nach Anspruch 1, wobei x 0 ist.

4. Verbindung zum Gebrauch nach Anspruch 3, wobei R³ ein optional substituiertes Aryl ist.

5. Verbindung zum Gebrauch nach Anspruch 4, wobei X¹ NH ist.

6. Verbindung zum Gebrauch nach Anspruch 5, wobei die Arylgruppe einen Substituenten aufweist.

7. Verbindung zum Gebrauch nach Anspruch 6, wobei der Substituent ein Anteil, ausgewählt aus der Gruppe bestehend aus Halogen, OH, O(C₁-C₄)alkyl, NH(C₁-C₄)alkyl, O(C₁-C₄)fluoralkyl und N[(C₁-C₄)alkyl]₂, ist.

8. Verbindung zum Gebrauch nach Anspruch 7, wobei der Substituent OH ist.

9. Verbindung zum Gebrauch nach Anspruch 1, wobei die Verbindung Folgende ist: 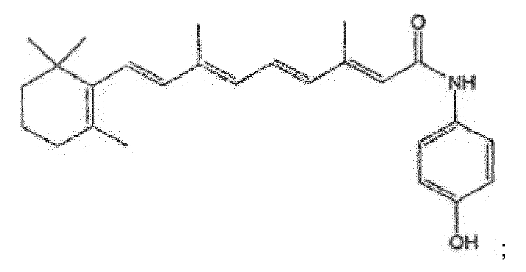 oder ein pharmazeutisch unbedenkliches Solvat davon.

10. Verbindung zum Gebrauch nach Anspruch 1, wobei die Verbindung 4-Hydroxyphenylretinamid oder ein pharmazeutisch unbedenkliches Salz oder Solvat davon ist.

11. Verbindung zum Gebrauch nach Anspruch 1, wobei die wirksame Menge der Verbindung dem Menschen systemisch verabreicht wird.

12. Verbindung zum Gebrauch nach Anspruch 11, wobei die wirksame Menge der Verbindung dem Menschen oral verabreicht wird, etwa als mehrfache Verabreichung der wirksamen Menge der Verbindung.

13. Verbindung zum Gebrauch nach Anspruch 1, ferner umfassend das Verabreichen von wenigstens einem weiteren Mittel, ausgewählt aus der Gruppe bestehend aus einem Induktor für Stickoxidproduktion, einem entzündungshemmenden Mittel, einem physiologisch unbedenklichen Antioxidans, einem physiologisch unbedenklichen Mineral, einem negativ geladenen Phospholipid, einem Carotinoid, einem Statin, einem antiangiogenischen Medikament, einem Matrixmetalloproteinasehemmer, Resveratrol und anderen Transstilbenverbindungen und 13-Cisretinolsäure.

14. Verbindung zum Gebrauch nach Anspruch 1, die 4-Oxofenretinid ist.

## Revendications

1. Composé de formule (II) à utiliser dans le traitement du glaucome, de l'hypertension oculaire ou de l'une de leurs combinaisons, chez un humain, dans lequel le composé de formule (II) a la structure : 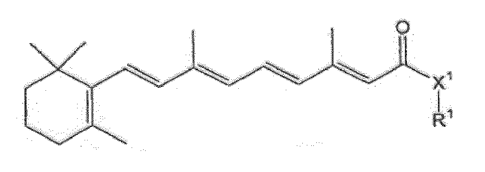 dans lequel X¹ est choisi dans le groupe consistant en NR², O, S, CHR² ; R¹ est (CHR²)ₓ-L¹-R³, dans lequel x vaut 0, 1, 2, ou 3 ; L¹ est une simple liaison ou -C(O)- ; R² est un fragment choisi dans le groupe consistant en H, alkyle (en C₁ à C₄), F, fluoroalkyle (en C₁ à C₄), alcoxy (en C₁ à C₄), -C(O)OH,
-C(O)-NH₂, -alkyl (en C₁ à C₄) amine, -C(O)-alkyle (en C₁ à C₄), -C(O)-fluoroalkyle (en C₁ à C₄), -C(O)-alkyl (en C₁ à C₄) amine, et -C(O)-alcoxy(en C₁ à C₄); et R³ est H ou un fragment choisi dans le groupe consistant en alcényle (en C₂ à C₇), alcynyle (en C₂ à C₇), aryle, cycloalkyle (en C₃ à C₇), cycloalcényle (en C₅ à C₇), et un hétérocycle, facultativement substitué par 1 à 3 substituants choisis indépendamment ; à condition que R³ ne soit pas H lorsqu'à la fois x vaut 0 et L¹ est une simple liaison ; un ou sel ou solvate pharmaceutiquement acceptable de celui-ci.

2. Composé à utiliser selon la revendication 1, dans lequel le composé est le 4-méthoxyphénylrétinamide.

3. Composé à utiliser selon la revendication 1, dans lequel x vaut 0.

4. Composé à utiliser selon la revendication 3, dans lequel R³ est un aryle facultativement substitué.

5. Composé à utiliser selon la revendication 4, dans lequel X¹ est NH.

6. Composé à utiliser selon la revendication 5, dans lequel le groupe aryle a un substituant.

7. Composé à utiliser selon la revendication 6, dans lequel le substituant est un fragment choisi dans le groupe consistant en halogène, OH, O-alkyle (en C₁ à C₄), NH-alkyle (en C₁ à C₄), O-fluoroalkyle (en C₁ à C₄), et N[alkyle (en C₁ à C₄)]₂.

8. Composé à utiliser selon la revendication 7, dans lequel le substituant est OH.

9. Composé à utiliser selon la revendication 1, dans lequel le composé est 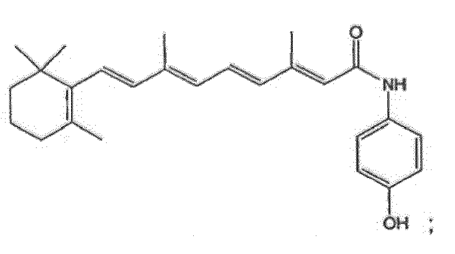 ou un solvate pharmaceutiquement acceptable de celui-ci.

10. Composé à utiliser selon la revendication 1, dans lequel le composé est le 4-hydroxyphénylrétinamide ; ou un sel ou solvate pharmaceutiquement acceptable de celui-ci.

11. Composé à utiliser selon la revendication 1, dans lequel la quantité efficace du composé est administrée à l'humain par voie systémique.

12. Composé à utiliser selon la revendication 11, dans lequel la quantité efficace du composé est administrée à l'humain par voir orale, par exemple sous forme d'administrations multiples de la quantité efficace du composé.

13. Composé à utiliser selon la revendication 1, comprenant en outre l'administration d'au moins un agent additionnel choisi dans le groupe consistant en un inducteur de production d'oxyde nitrique, un agent anti-inflammatoire, un antioxydant physiologiquement acceptable, un minéral physiologiquement acceptable, un phospholipide chargé négativement, un caroténoïde, une statine, un médicament antiangiogénique, un inhibiteur de métalloprotéinase matricielle, le resvératrol et d'autres composés de trans-stilbène, et l'acide 13-cis-rétinoïque.

14. Composé à utiliser selon la revendication 1, qui est le 4-oxo-fenrétinide.
